# EUROPEAN PATENT APPLICATION

(11) **EP 2 535 709 A2**
(43) Date of publication of application: **19.12.2012**
(21) Application number: 11742422.6
(22) Date of filing: 08.02.2011
(51) Int. Cl.: G01N 33/483, G01N 27/00, G06T 7/60, G01N 33/48

(54) **METHOD FOR IDENTIFYING MODULATORS THAT MODULATE PHYSIOLOGICAL FUNCTIONS OF INTRACELLULAR COMPONENTS WITHIN A CELL**

(30) Priority: 12.02.2010 KR 20100013372
(71) Applicant: Mediscov Inc., Yuseong-gu, Daejeon 305-811 (KR)
(72) Inventor: KIM, Dae Joong, Daejeon 305-761 (KR)
(74) Representative: Isarpatent
(86) International application number: PCT/KR2011/000790
(87) International publication number: WO 2011/099730

(57) **Abstract**

A method for detecting a modulator that can regulate biological and/or physiological activity of a cellular component is provided. The method can detect a modulator, after determining whether there is a competitive reaction between a reference substance and a modulator toward the cellular component in a cell by imaging a magnetic pattern magnetized in a direction of a magnetic line of force and a pattern of a label bound to the cellular component. The method has a benefit that since the reaction of a cellular component with a reference substance and a modulator such as a drug occurs in a cell, the competition reaction between the reference substance and the modulator toward the cellular component can reflect the real metabolism occurred in a live cell. It can also allow the result of interaction between the cellular component and the modulator such as a drug to be visualized and to be identified directly in a live cell. Additionally, the method has advantages to screen many modulators using a few reference substances and to detect the functions of the modulator in a cell, e.g., its efficacy without modifying the modulator by introducing the reference substance.

## Description

### FIELD OF THE INVENTION

The present invention relates to a method for detecting a modulator that can regulate biological and/or physiological activity of a cellular component in a cell, more particularly to a method for detecting whether there is a competitive reaction between a reference substance and a modulator toward the cellular component in a cell by imaging a label capable of showing a pattern aligned with a direction of a magnetic line of force.

### BACKGROUND OF THE INVENTION

The biological and/or physiological functions of a cell are regulated and maintained by a variety of cellular components in a cell, such as macromolecules, including proteins, nucleic acids, polysaccharides and lipids, and small molecules including amino acids, nucleotides, phosphoric acids, vitamins, amines and other organic compounds. When the normal biological and/or physiological functions of cellular components are disturbed or inhibited, some diseases may occur. Many efforts have been made to restore cellular activities and to treat diseases resulting from the abnormal functions of cellular components by using a modulator in biotechnological and pharmaceutical field.

To develop new modulators, some conventional methods, such as *in vitro* binding assay, bacteriophage display technology (see U.S. Patent Publication Nos. US2005/0009099A1 and US2008/0058219A1), or affinity chromatography (see PCT Publication No. WO 2006/134056A1), have been widely used.

In the aforesaid conventional methods, however, human-derived proteins had to be modified to allow them to be expressed in *E.coli* and obtained from *E.coli,* and the modified proteins are typically smaller in their size than the original ones. Since the human-derived proteins were obtained using *E.coli* system, the properties and functions of the proteins may be different from those of authentic proteins. Moreover, conventional target-based drug screenings are typically performed in an artificial buffer system, which makes the reaction of a protein target and a drug different from real cellular events. Especially, there is a problem when the conventional methods that target protein-drug interaction are indirectly identified through additional experimental processes that result in a high rate of false positives. Furthermore, in the conventional methods, the results are highly dependent on experimental conditions, such as cell lysis buffer, binding buffer for drug-protein binding, etc. Therefore, the reproducibility of the experiment according to the conventional methods may be decreased, depending upon external environment or technical errors resulting from the complexity of the experimental procedures.

In conclusion, conventional methods, which are performed *in vitro,* have difficulty in predicting the efficacy of a drug in a cell and/or *in vivo* in the drug development. Accordingly, there is a need in the art for a method for efficiently screening a modulator capable of regulating the biological and/or physiological activity of a cellular component in a cell by its binding with the cellular component.

In order to solve the aforesaid problems of the conventional methods, the present invention have developed a method for identifying a modulator that can regulate biological and/or physiological activity of a cellular component in a cell, by comparing a competitive response, between a reference substance (substance known to regulate a certain biological and/or physiological activity of cellular components) and modulators of the invention, toward the cellular components in a cell, by imaging a label capable of showing a pattern aligned with a direction of a magnetic line of force.

### SUMMARY OF THE INVENTION

The object of the invention is to provide a method for detecting whether there is a competitive reaction between a reference substance and a modulator toward a cellular component in a cell by imaging a magnetic pattern of magnetic substances magnetized in a direction of a magnetic line of force and a pattern of a label bound to the cellular component.

Further, the object of the invention is to provide a method for detecting a modulator that can regulate biological and/or physiological activity of a cellular component, after determining whether there is a competitive reaction between a reference substance and a modulator toward the cellular component in a cell, by imaging a magnetic pattern of magnetic substances magnetized in a direction of a magnetic line of force and a pattern of a label bound to the cellular component.

First of all, the terms used herein will be explained as follows.

The term "cellular component" refers to any biological substance, which regulates biological and/or physiological activities in a cell. For example, it may include macromolecules like proteins, nucleic acids, polysaccharides and lipids, and small molecules like amino acids, nucleotides, phosphoric acids, vitamins, amines and other organic compounds, which may be regarded as ones related to diseases or physiological metabolisms in a body.

The term "modulator" refers to a substance that can normalize the abnormal function of a cellular component whose normal biological and/or physiological function was disturbed or inhibited in a cell. It can generally normalize the physiological metabolisms and have potential to treat a certain disease. For instance, small molecules such as drugs, proteins such as antibodies, hormones or growth factors, protein domains, protein motifs, and peptides can act as a modulator.

The term "reference substance" refers to any substance known to regulate a certain biological and/or physiological activity of a cellular component, and can compete with the modulator toward the cellular component as stated above. It is also a potent substance known to normalize the physiological metabolism or to treat a certain disease by normalizing the cellular metabolisms in a body. For instance, small molecules such as drugs, proteins such as antibodies, hormones or growth factors, protein domains, protein motifs, and peptides can be also be a reference substance.

The term "labeling substance or label" refers to a substance that can image whether there is a competition reaction between the reference substance and the modulator toward the cellular component, and can visualize the competition reaction by a pattern. It may include radioactive materials, fluorescent or emitting materials. For example, a fluorescent protein such as EGFP (Enhanced Green Fluorescent Protein), GFP (Green Fluorescent Protein), YFP (Yellow Fluorescent Protein), CFP (Cyan Fluorescent Protein) or RFP (Red Fluorescent Protein) can be used to identify the existence, location and overall pattern of the cellular component.

Meanwhile, the term "surface modification" described herein means modifying or altering the surface of one substance to facilitate its binding to the other substance without changing the fundamental physical properties of the substance subjected to be modified. For example, it means, but not limited hereto, that an antigen for binding to a particular antibody or a receptor for ligand binding is attached to the surface of a particle such as a magnetic substance.

Further, the term "associating or associated" described herein means binding two subject substances to each other directly or indirectly. For example, it means, but not limited hereto, that when the surface of a particle is modified by an antigen or a receptor, the antigen or the receptor on the surface of the particle allows the particle to be bound to an antibody or a ligand corresponding to the antigen or the receptor, respectively.

Hereinafter, the constitution of the invention will be described using the terms as stated above.

In one embodiment, the present invention provides a method for detecting a modulator that can regulate biological and/or physiological activity of a cellular component in a cell comprising: preparing a plurality of magnetic substances magnetized in a direction of a magnetic line of force by a magnetic field, at least one of the magnetic substances being configured into a nanoparticle and the surface of at least one of the magnetic substances being modified; associating a reference substance capable of regulating the activity of the cellular component with the magnetic substance and then introducing the reference substance and the magnetic substance into a cell, a plurality of the magnetic substances associated with the reference substances being introduced to each cell; associating the cellular component to be bound to the reference substance with a label capable of imaging a pattern of the magnetic substances, and introducing the cellular component and the label into the cell; introducing a modulator into the cell, the modulator being tested to determine whether it competes with the reference substance toward the cellular component; allowing a bundle of the magnetic lines of force to pass through the cell in a direction by applying a magnetic field to the cell; aligning a plurality of the magnetic substances in the cell with the direction of the magnetic lines of force by the applied magnetic field; and identifying a pattern in which a plurality of the magnetic substances are aligned with the direction of the magnetic lines of force and a pattern of the label, the patterns being imaged.

In one embodiment of the present invention, the method further comprises identifying the imaged patterns of the magnetic substances and the label, and whether the imaged patterns are co-localized with each other or not.

In another embodiment, the present invention comprises a method wherein the modulator binds to the cellular component, and the imaged pattern of the magnetic substances is not co-localized with the imaged pattern of the label, when the modulator competes with the reference substance for binding to the cellular component. If the imaged patterns are not co-localized, therefore, it can be considered that the modulator binds to the cellular component.

In a further embodiment, the present invention comprises a method wherein the reference substance binds to the cellular component, and the imaged pattern of the magnetic substances is co-localized with the imaged pattern of the label, when the modulator does not compete with the reference substance for binding to the cellular component. If the imaged patterns are co-localized, therefore, it can be considered that the modulator does not bind to the cellular component.

It is preferable that the method further comprises introducing both the reference substance and the magnetic substance into a live cell and then exposing both the reference substance and the magnetic substance to cytoplasm of the live cell. More preferably, the modulator may be introduced into the live cell before both the reference substance and the magnetic substance are exposed to the cytoplasm of the live cell.

In one embodiment, the present invention comprises a method wherein the cellular component, the reference substance and/or the modulator may include macromolecules such as proteins, nucleic acids, polysaccharides and lipids, and small molecules such as amino acids, nucleotides, phosphoric acids, vitamins, amines and other organic compounds.

In another embodiment of the present invention comprises a method which can remove the property of a cellular component binding to the cell membrane, if the cellular component has a property binding to the cell membrane. In this case, the failure of imaging the pattern of the magnetic substances by the label after applying a magnetic field, which may be resulted from the labeled cellular component fixed to the cell membrane, can be prevented.

In another embodiment, the method of the present invention comprises removing the property of the cellular component capable of binding to the cell membrane by replacing the second amino acid of the protein, glycine with alanine, if the cellular component is BLK, FGR, LYN, SRC, or YES1 protein.

In addition, in another embodiment of the present invention, if the cellular component is KIT protein, the property of the cellular component capable of binding to the cell membrane can be removed by providing only the region of the amino acids 545 to 976 of the KIT protein into a live cell.

In another embodiment of the present invention, if the cellular component is ABL1 protein, the property of the cellular component capable of binding to the cell membrane can be removed by providing only the catalytic domain of ABL1 into a live cell.

The present invention can detect whether there is a competitive reaction between a reference substance and a modulator toward a cellular component in a cell by imaging a magnetic pattern of magnetic substances magnetized in a direction of a magnetic line of force and a pattern of a label bound to the cellular component, and it can also detect a modulator that can regulate biological and/or physiological activity of the cellular component by determining whether there is a competitive reaction between the reference substance and the modulator toward the cellular component in a cell.

According to the method of the present invention, since there is no need to purify or modify a cellular component such as a protein, the cellular component has its original form and function in a cell. Furthermore, unlike conventional screening methods that can detect the efficacy of drugs using purified cellular components, the present invention can monitor the selectivity of a cellular component for a modulator in a cell, which gives a benefit to precisely predict the reaction of the modulator such as a drug in a human body.

Moreover, according to the method of the present invention, since the reaction of the cellular component with the reference substance and the modulator such as a drug occurs in a cell, the competition reaction between the reference substance and the modulator toward the cellular component can reflect the real metabolism occurred in a live cell. Also, the present invention allows the result of interaction between the cellular component and the modulator such as a drug to be visualized and to be identified directly in a live cell.

Furthermore, since the method uses the competition reaction between the reference substance and the modulator toward the cellular component, the result of interaction between the cellular component and the modulator can be easily confirmed. For example, if the imaged pattern of the magnetic substances is formed, it is determined that the reaction between the cellular component and the modulator is negative, whereas if the imaged pattern of the magnetic substances is not formed, it is determined that the reaction between the cellular component and the modulator is positive.

This further provides advantages to screen many modulators using a few reference substances and to detect the functions of the modulator in a cell, e.g., its efficacy without modifying the modulator by introducing the reference substance.

### BRIEF DESCRIPTION OF THE DRAWINGS

These and other objects and features of the present invention will be apparent to those skilled in the art upon reading and understanding the following detailed description of the preferred embodiment of the invention with reference to the following drawings in which:

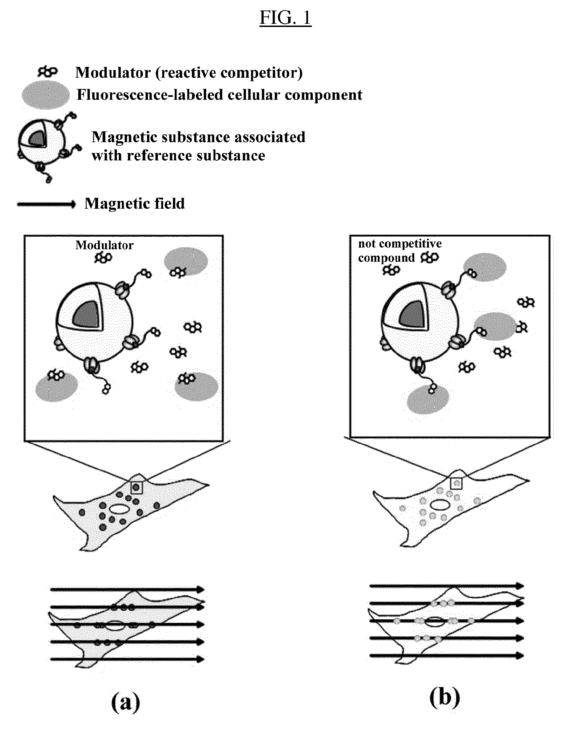

Figure 1 contains a schematic diagram showing the concept of identifying a small molecular compound (a reactive competitor) capable of binding to a cellular component using the competitive reaction.

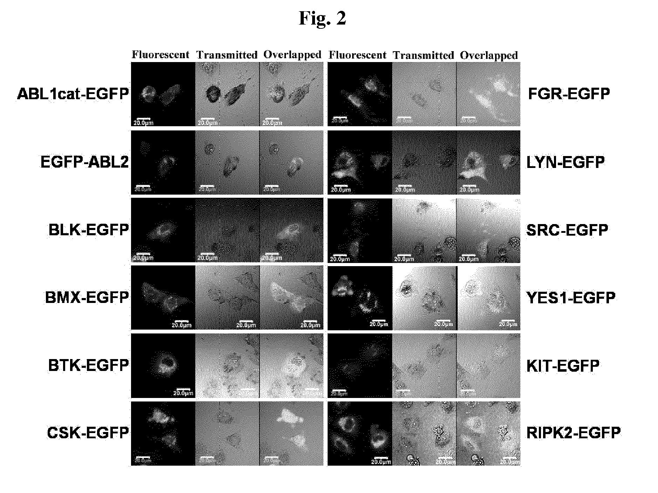

Figure 2 contains pictures taken from a fluorescent microscope showing a fluorescent pattern that is overlapped (co-localized) with a pattern of induced magnetization formed by magnetic substances in a direction of a magnetic field when a fluorescence-labeled cellular component binds to a reference substance associated with a surface-modified magnetic substance.

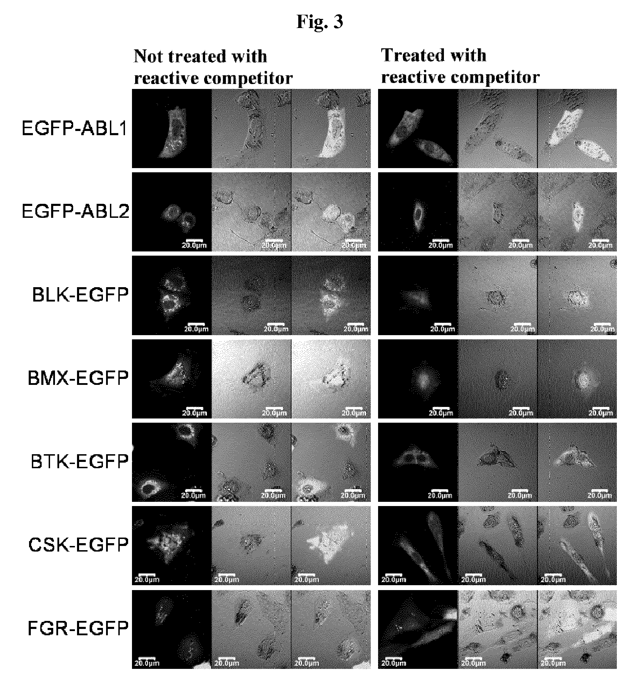

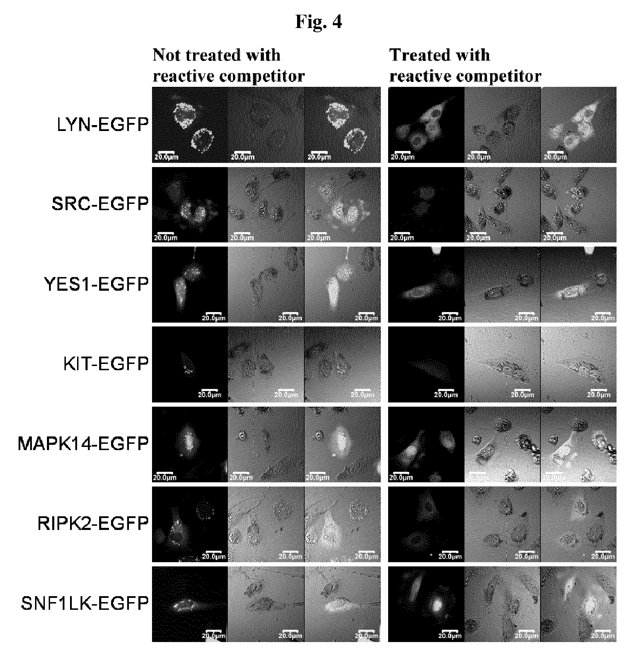

Figures 3 and 4 contain pictures taken from a fluorescent microscope showing whether a reactive competitor binds to a cellular component depending upon treating or not treating with the reactive competitor of unbiotinylated dasatinib. When not treating with a reactive competitor, it is confirmed that a fluorescent pattern was overlapped (co-localized) with a pattern of induced magnetization formed by magnetic substances in a direction of a magnetic field since a dasatinib-biotin associated with a surface-modified magnetic substance binds to a fluorescence-labeled cellular component. In contrast, when treating with a reactive competitor, it is confirmed that a pattern of induced magnetization was formed by magnetic substances in a direction of a magnetic field but a fluorescent pattern overlapped with the pattern of the induced magnetization was not observed since a reactive competitor binds to a cellular component.

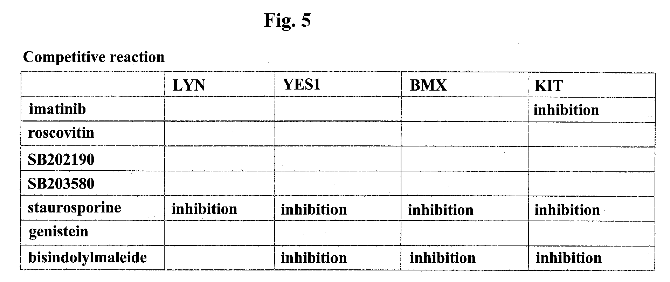

Figure 5 contains a table showing the result of screening modulators (small molecular compounds) capable of binding to a cellular component by the competition reaction. In at least two independent experiments conducted in different days, small molecular compounds that have been confirmed as modulators capable of binding to the cellular component are represented by "inhibition".

### DETAILED DESCRIPTION OF THE PREFERRED EMBODIMENTS

Practical and presently preferred embodiments of the present invention are illustrated more clearly as shown in the following examples. However, it should be appreciated that those skilled in the art, on consideration of this disclosure, may make modifications and improvements within the spirit and scope of the present invention. References cited in the specification are incorporated into the present invention.

### Example 1. Identifying the binding of a reference substance with a cellular component

In this embodiment of the present invention, it is determined whether a magnetization pattern of a plurality of magnetic substances formed by applying a magnetic field can be imaged in accordance with a fluorescent material as a label, and whether the pattern of the magnetic substances in a direction of a magnetic line of force is overlapped (co-localized) with the fluorescent pattern of the label, when the pattern of the magnetic substances (surface-modified and associated with a reference substance) magnetized in a direction of a magnetic line of force and the pattern of the label bound to a cellular component are imaged. For example, a list of kinase includes, but not limited to hereto, a cellular component.

To confirm whether a reference substance binds to a cellular component in a cell, the following experiment was performed. To begin with, the cellular component that is thought to interact with the selected reference substance was expressed while bound to a fluorescent protein in a cell. After a surface-modified magnetic substance associated with the reference substance was introduced into the cell, it was observed whether the pattern of the magnetic substances in a direction of a magnetic line of force was overlapped with the fluorescent pattern of the fluorescent protein, by binding the reference substance with the cellular component when applying a magnetic field.

### (1) Reference substances

A therapeutic agent for chronic myeloid leukemia, dasatinib was selected as a reference substance which is known to regulate biological and/or physiological activity of a cellular component [Lombardo, L. J., Lee, F. Y., Chen, P., et al. Discovery of N-(2-chloro-6-methyl-phenyl)-2-(6-(4-(2-hydroxyethyl)-piperazin-1-yl)-2-methylpyrimidin-4-ylamino)thiazole-5-carboxamide (BMS-354825), a dual Src/Abl kinase inhibitor with potent antitumor activity in preclinical assays. J. Med. Chem. 47, 6658-6661 (2004); Shah, N. P., Tran, C., Lee, F. Y. Chen, P., Norris, D., Sawyers, C. L. Oerriding imatinib resistance with a novel ABL kinase inhibitor. Science 305, 399-401 (2004)].

Dasatinib-biotin was synthesized to allow dasatinib to be bound with a magnetic particle whose surface was modified with streptavidin. The synthesis of dasatinib-biotin was performed according to the methods described in the Korean Patent Application No. 10-2008-0111326 (filed on November 10, 2008 by the inventors of the present invention).

For this example, dasatinib was used as a reference substance; however, the invention is not limited hereto. It is easily understood by an ordinarily skilled person in the art that a variety of small molecular compounds that can be attached to a magnetic substance and introduced into a cell, proteins such as antibodies, hormones and growth factors, protein domains, protein motifs, and peptides can be used as a reference substance.

### (2) Cellular components

For cellular components regulating biological and/or physiological activities in a cell, ABL1, ABL2, BLK, BMX, BTK, CSK, FGR, LYN, SRC, YES1, KIT, MAPK14, RIPK2, and SNF1LK were used to interact with dasatinib. The DNA templates for PCR were purchased from Open Biosystems, or cDNA library DNAs were used. To express a protein coding each gene in a human-derived cell in a fused form with a fluorescent protein, the ORF without the stop codon was amplified for each gene by PCR and then cloned into an expression vector, according to the methods described in the Korean Patent Application No. 10-2008-0111326 (filed on November 10, 2008).

Meanwhile, the second amino acid of proteins such as BLK, FGR, LYN, SRC, and YES1, "glycine" is known to bind to the cell membrane through myristoylation [McCabe, J. B. & Berthiaume, L. G. (1999) Functional roles for fatty acylated amino-terminal domains in subcellular localization. Mol. Biol. Cell 10, 3771]. Therefore, in this embodiment of the present invention, the second amino acid was replaced with alanine and then the respective protein was cloned to prevent the myristoylation of the proteins [McCabe, J. B. & Berthiaume, L. G. (1999) Functional roles for fatty acylated amino-terminal domains in subcellular localization. Mol. Biol. Cell 10, 3771].

Also, KIT is a membrane receptor kinase having a single transmembrane domain [Yarden, et al. (1987) Human proto-oncogene c-kit: a new cell surface receptor tyrosine kinase for an unidentified ligand. EMBO J. 6, 3341]. Thus, in this embodiment of the present invention, in order to remove the transmembrane domain of KIT, only the region of the amino acids 545 to 976 was amplified by PCR to make an expression vector.

In case of ABL1, the catalytic domain of ABL1 was cloned to produce the ABL1cat expression vector.

Furthermore, the completed expression vectors were identified by the end sequencing. The following Table 1 shows gene names, GenBank Access Nos. and properties of expressed protein kinases used as cellular components in this embodiment of the present invention.

Meanwhile, cellular components available in this invention are not limited to the protein kinases as mentioned in Table 1; however the cellular components may include a broad range of materials that regulate biological and/or physiological activities in a cell, for example, materials which are regarded to be associated with physiological metabolisms and diseases in a body.

**Table 1**

| Gene name | GenBank Acc. No. | Remark |
|---|---|---|
| ABL1 | NM_007313.2 | isoform b |
| ABL1cat | NM_007313.2 | amino acids 247∼519 |
| ABL2 | NM_007314.1 | isoform c |
| BLK | NM_001715 | G2A |
| BMX | NM_001721.6 | |
| BTK | NN_000061 | |
| CSK | NM_004383.1 | |
| FGR | NM_005248 | G2A |
| LYN | NM_001111097.1 | G2A |
| SRC | NM_198291 | G2A |
| YES1 | NM_005433 | G2A |
| KITc | BC071593 | amino acids 545-976 |
| MAPK14 | NM_001315 | |
| RIPK2 | NM_003821 | |
| SNF1LK | BC038504 | |

### (3) Identifying the binding of a reference substance with a cellular component

HeLa cells were cultured in a 96-well plate at the concentration of 5,000 to 10,000 cells/well and then were transfected with the expression vector DNA prepared as described above. DNA transfection can be performed using the methods known in the art, such as lipofectamine (purchased from Invitrogen) or Fugene 6 (purchased from Roche).

Next, magnetic substances coated with dasatinib-biotin (dasatinib-magnetic substances) were introduced into the cells transfected with a DNA using the methods known in the art [see Josepthson, et al. (1999) High-efficiency intracellular magnetic labeling with novel superparamagnetic-Tat peptide conjugate. Bioconjug. Chem. 10, 186; Derfus, et al. (2004) Intracellular delivery of quantum dots for live cell labeling and organelle tracking. Adv. Mater. 16, 961; Frank, et al. (2003) Clinically applicable labeling of mammalian and stem cells by combining superparamagnetic iron oxides and transfection agents. Radiology 228, 480; Kim, et al. (2008) In situ monitoring of bindings between dasatinib and its target protein kinases using magnetic nanoparticles in live cells. J. Am. Chem. Soc. 130, 16466].

The magnetic substance used to be coated by dasatinib-biotin may be a superparamagnetic particle conjugated with streptavidin (streptavidin-magnetic particle). This streptavidin-magnetic particle can be purchased or made by the methods known in the art [Molday, et al. Immunospecific ferromagnetic iron-dextran reagents for the labeling and magnetic separation of cells. J. Immunol. Meth. (1982) 52, 353-367; March, et al., A Simplified method for cyanogen bromide activation of agarose for affinity chromatography. Anal. Biochem. (1974) 60, 149-152; Cuatrecasas, P. Protein purification by affinity chromatography. J. Biol. Chem. (1970) 245, 3059-3065; U.S. Patent No. 5,665,582; U.S. Patent Publication No. 2003/0092029A1].

On the next day, in dasatinib-magnetic substance-treated cells, the binding of the reference substance (dasatinib) with a cellular component was performed by exposing the dasatinib-magnetic substance to the cytoplasm according to the methods known in the art [see Kim, et al. (2008) In situ monitoring of bindings between dasatinib and its target protein kinases using magnetic nanoparticles in live cells. J. Am. Chem. Soc. 130, 16466].

According to the methods known in the art, a pattern of induced magnetization of the magnetic substances associated with the reference substances was formed by applying a magnetic field [see Korean Patent No. 10-0792594; Korean Patent No. 10-0862368; U.S. Patent No. 4,247,398; Melville, D., F. Paul, & S. Roath (1975) Direct magnetic separation of red cells from whole blood. Nature 255: 706]. Under the magnetic field, the cells were fixed and observed using a microscope according to the methods known in the art [see Korean Patent Application No. 10-2008-0111326; Kim, et al. (2008) In situ monitoring of bindings between dasatinib and its target protein kinases using magnetic nanoparticles in live cells. J. Am. Chem. Soc. 130, 16466].

In this embodiment of the present invention, Olympus fluorescence microscope, FV1000, equipped with the object lens of Uplan Apo 40X/0.85 was used to obtain fluorescent and transmitted images in the cells. The result is illustrated in Figure 2. As shown in Figure 2, it was observed that when imaging the pattern of the magnetic substances (associated with dasatinib of the reference substance) magnetized in a direction of a magnetic line of force and the fluorescent pattern of EGFP associated with the kinase of the cellular component, the fluorescent pattern of EGFP was formed in a magnetized direction, i.e., in a direction of a magnetic line of force.

As illustrated in Figure 2, as a result of identifying whether the reference substance "dasatinib" and the kinases of the cellular components interact with each other, it was confirmed that kinase-EGFP fusion proteins expressed in the cells such as ABL1cat-EGFP, EGFP-ABL2, BLK-EGFP, BMX-EGFP, BTK-EGFP, CSK-EGFP, FGR-EGFP, LYN-EGFP, SRC-EGFP, YES1-EGFP, KIT-EGFP, and RIPK2-EGFP showed the fluorescent patterns aligned in a magnetized direction, i.e., in a direction of a magnetic line of force, with being bound to the dasatinib-magnetic particles. This fluorescent pattern appeared to be overlapped with the pattern of magnetic substances observed in the image of transmitted light. This result demonstrates that the fluorescent material EGFP has been accurately labeled on the magnetic particle linked with dasatinib-kinase to image the magnetized pattern.

In other words, the present invention can easily identify the binding between a reference substance and a cellular component in a cell by imaging the pattern of the magnetic substances (associated with dasatinib of the reference substance) magnetized in a direction of a magnetic line of force, and the fluorescent pattern of EGFP associated with the kinase of the cellular component. Therefore, the pictures of Figure 2 illustrate the binding of the dasatinib coated on the magnetic substance with kinases (ABL1, ABL2, BLK, BMX, BTK, CSK, FGR, LYN, SRC, YES1, KIT, and RIPK2).

### Example 2. Identifying the binding of a reactive competitor with a cellular component by competition reaction

Like Example 1 as described above, when a fluorescence-labeled cellular component binds to a reference substance associated with a surface-modified magnetic substance, a fluorescent pattern is formed and overlapped (co-localized) with a magnetization pattern of magnetic substances induced by an external magnetic field.

However, if there is a modulator (reactive competitor) that can bind to a particular part of a cellular component which is subjected to be bound to a reference substance in a cell, the modulator (reactive competitor) would bind to the cellular component and thus the interaction between the cellular component and the reference substance would be inhibited. Therefore, the cellular component could not bind to the reference substance associated with the surface-modified magnetic substance. As a result, a magnetization pattern of the magnetic substances would be induced in a magnetized direction, but a fluorescent pattern would not be formed anymore by the fluorescence labeling the cellular component in a magnetized direction (see Fig. 1a).

On the other hand, if a reactive competitor candidate does not bind a cellular component, the binding between a cellular component and a reference substance would be maintained, and a fluorescent pattern would be formed and overlapped with a magnetization pattern induced in a magnetized direction (see Fig. 1b). Accordingly, the observation on whether the fluorescent pattern was formed and overlapped with the magnetization pattern of the magnetic substances induced in a magnetized direction can give us important information as to whether the reactive competitor candidate was bound to the cellular component or not.

Using the competition reaction performed by a reactive competitor candidate as described above, the following experiment was performed to identify the interaction between a candidate and a cellular component.

First of all, a cellular component known to interact with a selected reference substance was expressed in a form bound with a fluorescent protein. When a surface-modified magnetic substance associated with the reference substance was introduced into a cell and treated with a reactive competitor, a fluorescent pattern overlapped with a pattern of magnetic substances in a direction of a magnetic line of force was not formed since the cellular component binds to the reactive competitor. In contrast, when not treating with a reactive competitor, a fluorescent pattern overlapped with a pattern of magnetic substances in a direction of a magnetic line of force was formed since the cellular component binds to the reference substance.

The cells that were introduced with a reference substance associated with a surface-modified magnetic substance and a fluorescence-labeled cellular component have been prepared as mentioned in Example 1. Before the dasatinib-magnetic substance was exposed to the cytoplasm of the cells, the cells were treated with 10 µM of unbiotinylated dasatinib as a reactive competitor for 30 minutes to 2 hours. After the dasatinib-magnetic substance was exposed to the cytoplasm, under a magnetic field, the cells were fixed and observed using a microscope as described in Example 1.

In this embodiment of the present invention, Olympus fluorescence microscope, FV1000, equipped with the object lens of Uplan Apo 40X/0.85 was used to obtain fluorescent and transmitted images in the cells. The results are illustrated in Figures 3 and 4.

As shown in Figures 3 and 4, it was observed that when not treating with the unbiotinylated dasatinib of the reactive competitor, a fluorescent pattern overlapped with a magnetization pattern of the magnetic substances induced in a direction of a magnetic field was formed since the fluorescence-labeled cellular components such as EGFP-ABL1, EGFP-ABL2, BLK-EGFP, BMX-EGFP, BTK-EGFP, CSK-EGFP, FGR-EGFP, LYN-EGFP, SRC-EGFP, YES1-EGFP, KIT-EGFP, MAPK14-EGFP, RIPK2-EGFP, SNF1LK-EGFP, etc. interacted with the dasatinib associated with the surface-modified magnetic substance. However, when treating with the unbiotinylated dasatinib of the reactive competitor, a fluorescent pattern overlapped with an induced magnetization pattern of the magnetic substances was not observed. This result demonstrates that the unbiotinylated dasatinib of the reactive competitor interacted with the fluorescence-labeled cellular component so that the fluorescence-labeled intracellular protein could not interact with the dasatinib of the reference substance associated with the surface-modified magnetic substance.

### Example 3. Screening for a modulator interacting with a cellular component

To screen a modulator (reactive competitor) such as small molecular compounds interacting with a cellular component using the competition reaction, the following experiment has been performed.

Small molecular compounds such as a modulator used in this example may include imatinib (CAS No.220127-57-1), roscovitin (CAS No.186692-46-6), SB202190 (CAS No. 152121-30-7), SB203580 (CAS No. 152121-47-6), staurosporine (CAS No. 62996-74-1), genistein (CAS No. 446-72-0), bisindolylmaleide I (CAS No. 176504-36-2), H-89 (CAS No. 127243-85-0), LY294002 (CAS No. 154447-36-6), PD98059 (CAS No. 167869-21-8), SU-1498 (Tyrphostin SU-1498), AG-1478 (Tyrphostin AG-1478)(CAS No. 175178-82-2), AG-490 (Tyrphostin AG-490)(CAS No. 134036-52-5), U-126 (CAS No. 109511-58-2), and HA-1077 (CAS No. 103745-39-7).

However, it is easily understood by an ordinarily skilled person in the art that a modulator is not limited to those as described above, but may include various candidates which could be introduced into a cell and could normalize the abnormal function of a cellular component whose normal biological and/or physiological function was disturbed or inhibited in a cell.

Meanwhile, the competitive reaction has been conducted as described in Example 2. Before the dasatinib-magnetic substance was exposed to the cytoplasm of the cells, the cells were treated with 10 µM of each small molecular compound, a modulator (reactive competitor) for 30 minutes to 2 hours. After the dasatinib-magnetic substance was exposed to the cytoplasm, under a magnetic field, the cells were fixed as described in Example 1 and observed using a microscope according to the methods as described in Example 2.

In at least two independent experiments conducted in different days, small molecular compounds (modulators) are represented by "inhibition" in Figure 5 if an induced magnetization pattern of the dasatinib-magnetic substances in a direction of a magnetic line of force was observed but a fluorescent pattern of the fluorescence-labeled cellular component was absolutely not observed. For example, as shown in Figure 5, it is confirmed that a cellular component, KIT interacts with a small molecular compound, imatinib as a modulator in a cell.

In conclusion, the method of the present invention has a benefit that since the reaction of the cellular component with the reference substance and the modulator such as a drug occurs in a cell, the competition reaction between the reference substance and the modulator toward the cellular component can reflect the real metabolism occurred in a live cell. It can also allow the result of interaction between the cellular component and the modulator such as a drug to be visualized and to be identified directly in a live cell. Additionally, the method of the present invention has advantages to screen many modulators using a few reference substances and to detect the functions of the modulator in a cell, e.g., its efficacy without modifying the modulator by introducing the reference substance.

## Claims

1. A method for detecting a modulator that can regulate biological and/or physiological activity of a cellular component in a cell, comprising:
preparing a plurality of magnetic substances magnetized in a direction of a magnetic line of force by a magnetic field, at least one of the magnetic substances being configured into a nanoparticle and the surface of at least one of the magnetic substances being modified;
associating a reference substance capable of regulating the activity of the cellular component with the magnetic substance and then introducing the reference substance and the magnetic substance into a cell, a plurality of the magnetic substances associated with the reference substances being introduced per one cell;
associating the cellular component subjected to be bound to the reference substance with a label capable of imaging a pattern of the magnetic substances, and providing the cellular component and the label into the cell;
introducing a modulator into the cell, the modulator being subjected to determine whether it competes with the reference substance toward the cellular component;
allowing a bundle of the magnetic lines of force to pass through the cell in a direction by applying a magnetic field to the cell;
aligning a plurality of the magnetic substances in the cell with the direction of the magnetic lines of force by the applied magnetic field; and
identifying a pattern in which a plurality of the magnetic substances are aligned with the direction of the magnetic lines of force and a pattern of the label, the patterns being imaged.

2. The method of claim 1, further comprises identifying the imaged patterns of the magnetic substances and the label, and whether the imaged patterns are co-localized with each other or not.

3. The method of claim 2, wherein when the modulator competes with the reference substance for binding to the cellular component, the modulator binds to the cellular component, and the imaged pattern of the magnetic substances is not co-localized with the imaged pattern of the label.

4. The method of claim 2, wherein when the modulator does not compete with the reference substance for binding to the cellular component, the reference substance binds to the cellular component, and the imaged pattern of the magnetic substances is co-localized with the imaged pattern of the label.

5. The method as claimed in any one of claim 1 to 4, further comprises providing both the reference substance and the magnetic substance into a live cell and then exposing both the reference substance and the magnetic substance to cytoplasm of the live cell.

6. The method of claim 5, wherein the modulator is introduced into the live cell before both the reference substance and the magnetic substance are exposed to the cytoplasm of the live cell.

7. The method as claimed in any one of claim 1 to 4, wherein if the cellular component has a property capable of binding to the cell membrane, the property of the cellular component capable of binding to the cell membrane is removed.

8. The method of claim 7, wherein if the cellular component is BLK, FGR, LYN, SRC, or YES1 protein, the property of the cellular component capable of binding to the cell membrane is removed by replacing the second amino acid of the protein, glycine with alanine.

9. The method of claim 7, wherein if the cellular component is KIT protein, the property of the cellular component capable of binding to the cell membrane is removed by providing only the region of the amino acids 545 to 976 of the KIT protein into a live cell.

10. The method of claim 7, wherein if the cellular component is ABL1 protein, the property of the cellular component capable of binding to the cell membrane is removed by providing only the catalytic domain of ABL1 into a live cell.
